(19) **Europäisches Patentamt**
**European Patent Office**
**Office européen des brevets**

(11) **EP 2 194 474 A1**

(12) **EUROPEAN PATENT APPLICATION**

(43) Date of publication:
**09.06.2010 Bulletin 2010/23**

(51) Int Cl.:
***G06F 19/00*** (2006.01)

(21) Application number: **08396017.9**

(22) Date of filing: **28.11.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **General Electric Company Schenectady, NY 12345 (US)**

(72) Inventors:
• **Heinonen, Erkki**
  **00750 Helsinki (FI)**
• **Häggblom, Tom**
  **01520 Vantaa (FI)**

(74) Representative: **Kanerva, Arto**
  **GE Healthcare Finland Oy**
  **P.O. Box 900**
  **00031 GE (FI)**

(54) **Drug delivery presentation and arrangement for drug delivery presentation**

(57) A drug delivery presentation is disclosed herein. The drug delivery presentation includes a display (1) for showing a concentration of at least two different types of drugs including a first drug and a second drug for a subject, and also for showing a coordinate system for a variable first drug concentration and a variable second drug concentration. The drug delivery presentation also includes an optimal therapy mixture area (4) presented on a coordinate system comprising a first border section (5) determining a minimum concentration mixture for the first drug and the second drug. The optimal therapy mixture area further comprising a second border section (6) determining a maximum concentration mixture for the first drug and the second drug. An arrangement for a drug delivery presentation is also disclosed.

Figure 1

**Description**

BACKGROUND OF THE INVENTION

This disclosure relates generally to a drug delivery presentation and an arrangement for drug delivery presentation when dosing at least two different types of drugs including a first drug and a second drug for a subject.

When anesthetizing patient, different anesthesia components, hypnosis, analgesia, and neuro-muscular blockade, needs to be considered. Of these neuro-muscular blockade has an own specific medication. Inhalation agents have both analgesic and hypnotic properties whereas intravenous (IV) drugs are more specific for these primary effects. But still the IV hypnosis drug involves as a secondary effect some analgesic properties and vice versa the analgesic drugs present some hypnotic properties as secondary effect. In addition to having these secondary effects the drugs may represent also synergistic characteristics, which means that the secondary effect of the drug amplifies the other drugs primary effect. In practice this appears in that for example even a small amount of the analgesic drug reduces significantly the amount of the hypnotic drug desired for a proper anesthesia even though the same amount of the analgesia drug alone would have no hypnotic effect. Respectively arising from this, an optimal anesthesia requires usually both the hypnotic and analgesic medication.

A drug effect on a patient correlates on the concentration of the drug at the effect site of the neural system. This concentration however cannot be measured directly, and therefore models determining the concentration from subject demographics and drug delivery rate history have been determined. IV delivery controllers use these models to automatically adjust the drug delivery in order to achieve and maintain a patient effect site drug concentration at given target level. The models are however based on statistical measurements in a specific limited population, and the concentrations required for adequate anesthesia may vary between subjects and during the course of surgical operation as a response to e.g. surgical stimuli. Therefore a clinician must adapt the target concentrations for every patient and adjust during the course of surgery. For this purpose the clinician follows measured patient physiological signals indicating the level of hypnosis and analgesia. In this adjustment the clinician must take into a consideration also other aspects like negative side effects of high drug concentrations, a cost of the drugs, and increasingly towards an end of a surgery a timing of a patient wake-up. Furthermore, some of these measured clinical parameters have a limited range of drug concentration they are responding correctly on the physiological effect they are used for. Beyond these limits the signals may not indicate the anesthesia state correctly.

Current IV drug delivery systems provide drug delivery displays where the drug effect site concentrations or determined from the model based effect site concentrations are presented over time. Alternatively the effect site concentrations are presented on XY coordinate system typically the analgesia drug concentration on X-axis and hypnosis drug concentration on the Y-axis. Also of this XY presentation a form where model based analgesia component represents the X-axis and hypnosis component represents the Y-axis including the synergistic effects between the drugs exists. These graphical presentations often incorporate also a statistical isobole information of the concentrations or mixtures where the anesthesia is sufficient for defined operation in 50% or 95% of subjects. All these displays however miss information of the true subject status based on physiological measurements. Also the other factors affecting on optimal therapy mixture is missing.

BRIEF DESCRIPTION OF THE INVENTION

The above-mentioned shortcomings, disadvantages and problems are addressed herein which will be understood by reading and understanding the following specification.

In an embodiment, a drug delivery presentation includes a display for showing a concentration of at least two different types of drugs including a first drug and a second drug for a subject and also for showing a coordinate system for a variable first drug concentration and a variable second drug concentration. The drug delivery presentation also includes an optimal therapy mixture area presented on a coordinate system comprising a first border section determining a minimum concentration mixture for the first drug and the second drug. The optimal therapy mixture area further comprising a second border section determining a maximum concentration mixture for the first drug and the second drug.

In another embodiment, a drug delivery presentation includes a display for showing a concentration of both a hypnotic drug and an analgesic drug for a subject, these drugs having at least one parallel physiological effect. The drug delivery presentation also includes a first axis on the display being vertical and a second axis on the display being horizontal and which axes are perpendicular to each other, one of these axis being for a variable hypnotic drug concentration and the other being for a variable analgesic drug concentration. The drug delivery presentation further includes an optimal therapy mixture area presented on a coordinate system formed of the first axis and the second axis and comprising a first border section determining a minimum concentration mixture for the hypnotic drug and the analgesic drug. The optimal therapy mixture area further comprising a second border section determining a maximum concentration mixture for the hypnotic drug and the analgesic drug.

In yet another embodiment an arrangement for a drug delivery presentation, when dosing at least two different types of drugs including a first drug and a second drug for a subject, includes a first dosing device for dosing the first drug, a second dosing device for dosing the second drug and a measurement unit for measuring at least one physiological parameter and creating a signal indicative of the at least one measured physiological parameter. The arrangement for the drug delivery presentation also includes a user interface for entering a subject demographic information and a processing unit for determining a display information based on the information received from the user interface and the signal received from the measurement unit. The arrangement for the drug delivery presentation further includes a display for receiving the display information from the processing unit for showing a concentration of at least the first drug and the second drug and for showing an optimal therapy mixture area comprising a first border section determining a minimum concentration mixture for the first drug and the second drug and which optimal therapy mixture area is presented on a coordinate system. The optimal therapy mixture area further comprising a second border section determining a maximum concentration mixture for the first drug and the second drug.

Various other features, objects, and advantages of the invention will be made apparent to those skilled in art from the accompanying drawings and detailed description thereof.

BRIEF DESCRIPTION OF THE DRAWINGS

Figure 1 shows a drug delivery presentation in accordance with an embodiment; and

Figure 2 shows an arrangement used with a drug delivery presentation of Figure 1.

DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

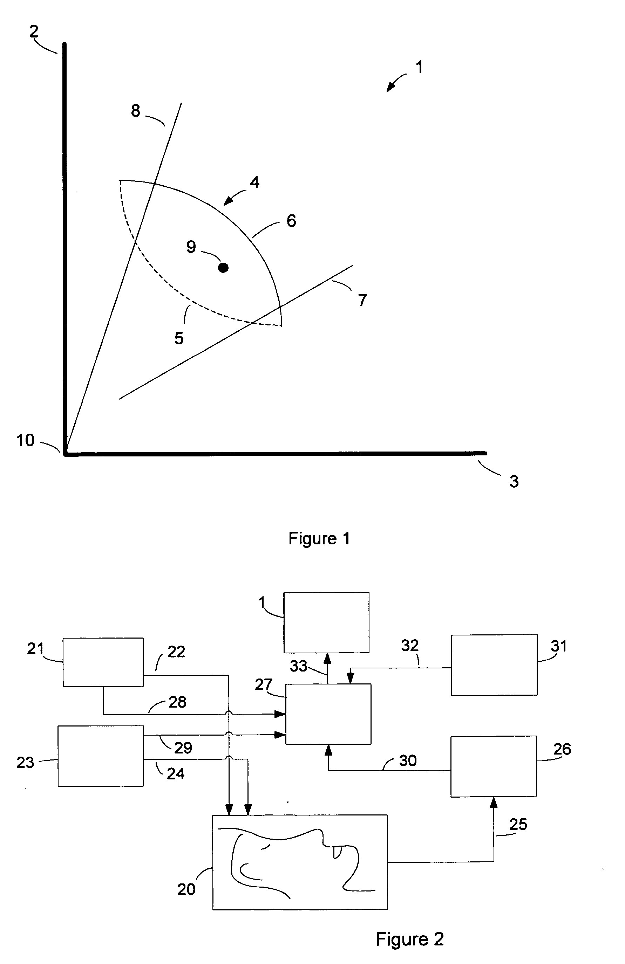

An embodiment of a drug delivery presentation on a display 1 as shown in Figure 1 exploits a first axis 2 and a second axis 3 and which the second axis is diverging from the first axis. Typically the first axis 2 is vertical and the second axis 3 is horizontal being perpendicular to each other such as known XY axes. The first axis 2 and the second axis 3 on a coordinate system have an origin 10. The display 1 shows the coordinate system formed of the first axis and the second axis and a concentration resulting from a dosage of at least two different types of drugs including a first drug and a second drug for a subject. Concentration values of both the first drug and the second drug are increasing along the axes from the origin 10.

Frequently these drugs have parallel physiological effects, which may mean that the physiological effect of the first drug must add into the parallel physiological effect of the second drug in which case this physiological effect is a side effect of the first drug and a main effect of the second drug. Also it is possible that these drugs have a synergistic effect on another drug's physiological effect amplifying this physiological effect. Further it is possible that the physiological effect of the second drug must add into the parallel physiological effect of the first drug in which case this physiological effect is a side effect of the second drug and a main effect of the first drug.

The drug delivery presentation in Figure 1 shows variable concentration values of a hypnotic drug, which is the first drug on the first axis 2 being vertical and an analgesic drug, which is the second drug on the second axis 3 being horizontal. An optimal therapy mixture area 4 for the first and second drugs is superimposed on this as presented in the Figure 1. This optimal therapy mixture area 4 may be based on at least one measured subject physiological parameter like electroencephalography (EEG), electromyography (EMG), plethysmography, blood pressure, or heart rate or an index based on electroencephalography, electromyography (EMG) or plethysmography.

Initially when beginning an anesthesia prior to any measurement information becomes available the optimal therapy mixture area 4 may be based on a subject demographic information, such as a length, weight, sex and age, and at least one known parallel physiological effect of those drugs. The optimal therapy mixture area 4 for the first and second drugs is comprised of a first border section 5 (dashed line) determining a minimum concentration mixture such as minimum effect site concentration mixture for the first and second drugs providing a desired physiological effect whereby with higher drug concentration than the first border section 5 the desired physiological effect is achieved and a second border section 6 (solid line) determining a maximum concentration mixture such as a maximum effect site concentration mixture for the first and second drugs beyond which the undesired physiological effects of the drug mixture are unacceptable whereby with a lower drug concentration than the second border section 6 the desired physiological effect is achieved.

The first border section 5 providing the desired physiological effect may be initially determined using information about the subject demographic information and at least one known parallel physiological effect. This is then modified or updated using the measured drug response. For a hypnosis effect a brain activity indicator signals derived form EEG signal provided by the measurement system may be used.

Determination of the minimum concentration mixture for the first and the second drugs corresponding to the first border section 5 for the desired physiological effect may use well known sigmoidal drug concentration-effect response, so called Hill, curve. This curve is characterized by maximum effect, concentration providing 50% of the maximum effect ($EC_{50}$), and steepness of the curve rise and has the formula of

$$I \qquad E = \frac{E_{max} \cdot C^y}{C^y + EC_{50}{}^y}$$

Where E= drug effect, $E_{max}$ maximum drug effect, C=a drug concentration for first and second drugs, and y is a factor determining the slope of the sigmoidal rise.

The established EEG and EMG indicators have an inversed scale where at the maximum drug effect the brain activity gets to zero whereas 100 indicates maximum activity. The $EC_{50}$ is the dose where the EEG index equals 50. In the inverted mode the EEG index ($I_{EEG}$) becomes as

$$II \qquad I_{EEG} = 100 \cdot \left(\frac{EC_{50}{}^y}{C^y + EC_{50}{}^y}\right)$$

Collecting series of data of measured $I_{EEG}$ and the model-based $C$ the factors $EC_{50}$ and $y$ can be solved. $y$ may also be regarded as constant or determined initially during induction of anesthesia when at least two $C$-$I$-pairs can be collected. During further anesthesia the factor $EC_{50}$ can be adjusted to compensate for changes in this relationship caused e.g. by changes in the secondary drug concentrations, or surgical procedures, using the further measured $I_{EEG}$ -$C$ pairs. The $EC_{50}$ may be changed by a user manually also proactively to respond known coming changes in a surgical stimuli. Such changes may be programmed on a control algorithm and fine-tuned manually according to the subject response. The algorithm may also learn from the earlier anesthesia phases the desired changes caused by forthcoming actions.

The maximum concentration mixture for the first and the second drugs corresponding to the second border section 6 may be initially determined using the concentration known harmful to the subject either during or after the anesthesia. During the anesthesia the second border section 6 is modified or updated individually if indicated by one or more of the measured physiological signals like EEG, heart rate and blood pressure. The relationship of the negative or undesired effect to the concentration can be expressed as well using the relationship I hereinbefore with coefficients fitted for the negative effects. Also here the $y$-factor can be regarded constant, and adjust the position of the sigmoidal curve on the concentration axis by adjusting the $EC_{50}$ for the negative effect. The net application area of the drug is defined as the difference between the positive and negative effects.

Whereas the EEG indexes represent good measurement of the level of hypnosis controlled by the first drug, similar clinically validated indicator is still missing on the analgesia medication although plethysmography has potency for indication of subject analgesic status. Also sudden HR and BP increases and EMG activity may indicate subject pain feeling. However, in the lack of undisputed measurements, the minimum concentration of the second drug control must in large extent be based on subject demographic information, subject health status, drug manufacturer's instructions, usage of other drugs having analgesic effect, and visual observation of the subject.

Furthermore, the analgesic medication needs to be administered pro-actively before forthcoming painful stimuli to avoid subject pain experience. For these purposes clinician must have means to manually manipulate the minimum required concentration characteristics. When subject sensitivity to drug medication is learned based on the measured subject signal, the stimulus and this sensitivity information is used further in other painful surgery phases.

Some clinical parameters like heart rate and blood pressure are good indicators of too high analgesic drug doses, and unacceptable decrease in these may be used to tune the parameters of the negative effects of the analgesic medication. The negative analgesic effects may also incorporate known post-operative hyper algesia caused by excessive dosages of analgesic drugs and other known undesired subject side-effects of high analgesic drug concentrations. Too high analgesic medication may also interfere with the EEG index measurements in a way that its indicator value to subject level of hypnosis may become compromised.

The positive and negative concentration-effect relationships determine the optimal therapy for single drug and single anesthesia effect. This determination is repeated for the other anesthesia effect providing optimal drug for the other anesthesia effect. Once this has been characterized for both of the hypnotic and analgesic effects, the mixture area optimal for both anesthesia effects, which is the optimal therapy mixture area 4 in Figure 1 can be determined. This determination may be done e.g. by multiplying the values describing the optimal mixture in respect to single anesthesia component. The product then represents an intensity map on analgesia drug concentration- hypnotic drug concentration coordinates. The higher the product value is the more optimal the mixture concentration in terms of both anesthesia effect is. This intensity map can be used to draw isoboles of constant effects. The optimal therapy mixture area 4 may be then determined as values higher than e.g. 90% of the maximum.

A further advantage of the method is in its provision of the relationship to the subject measurements. Once the measurements directly modify the concentration-effect relationship equation parameters, these modifications immediately affect the optimal concentration area through the use of the relationships as described. These relationships also provide the concentration required in order to achieve the desired physiological measured value. Thus, these values can be used to determine the optimal drug mixture providing the physiological signal value within the user given target.

The drugs involved may be administered intravenously but also inhalation agents may be used.

The drawn effect side concentration graph may represent also other information related to optimize the drug mixture. This could include a wake-up indicator 74 of Figure 1 to help a timing of a subject wake-up. Different drugs have different half times from the subject, and these half times may also prolong along the anesthesia. An optimization of the first and the second drug mixture may incorporate also a time to wake-up in a way that the effect of the delivered first and second drugs fades away at the same time. Such presentation could be an isochrone line the position of which varies during the course of anesthesia. At the end of anesthesia the used mixture may be selected from values where this indicator 7 meets the area of optimal delivery mixture.

Further information to optimize the first and the second drug mixture could be a drug cost indicator 8 showing the first and the second drug cost which is acceptable being in most cases preferably minimal. This line intersection with the optimal therapy mixture area 4 could be used in the early anesthesia to minimize the costs. The graphical presentation may show also a current first and second drug delivery mixture indicator 9, which may be a spot as shown in Figure 1 and the delivery history (not shown in Figure 1). In automatic drug delivery control modes also a user defined dosage rate envelope comprising minimum and maximum concentration for each drug between which the controller may adjust the concentration for the drugs can be presented as well.

An advantage of the described drug delivery presentation is in giving an individualized guidance of the desired change in the drug delivery. In the lack of such guidance clinician may easily be mislead by the complexity of the information and treat with medication changes the symptoms instead of the cause. For example, the subject experiencing a pain may cause an arousal that results increases in EEG index, which the clinician may treat by increasing the hypnosis medication. This may result to an undesired high concentration of the hypnosis drug, which may impose negative effects to the subject like a reduced blood pressure or heart rate. In the described case the correct action would instead have been to increase the analgesic medication to prevent the subject feeling the pain, and thus avoiding the arousal. The drug delivery presentation guides the clinician for the correct actions to maintain the optimal drug mixture and to avoid a distorted delivery profile in a form of undesired high concentrations of the individual drug. Furthermore, the drug delivery presentation also provides the synergetic effect from other medications in addition to the controlled ones affecting on the anesthesia entities in the form that e.g. when the hypnosis is affected by a premedication, which is fading away in time, the optimum drug concentration of the presented hypnosis drug will increase accordingly. Similar cross-compensation is acting also between changes in hypnotic and analgesic medications where large change in one is advised to be compensated with the other.

Further advantage of the method is that it provides an opportunity to further ease the clinician's work by automatically perform the actions in drug delivery in order to maintain the desired optimum anesthesia state. In such a closed loop drug delivery control system the clinician may set a target value for one or more measurement parameters, and the controller then automatically adjusts the drug delivery in order to preserve the measured value within the target. Such automatic system would also automatically compensate with drug medication changes in the patient state and responds also to changes in other drugs given and affecting to the anesthesia entity.

Figure 2 shows an arrangement for the drug delivery presentation. The first drug such as the hypnotic drug is dosed to the subject 20 by means of a first dosing device 21 and through a first channel 22 to bring the subject under the anesthesia and maintain the anesthesia. The second drug such as the analgesic drug is dosed to the subject 20 by means of a second dosing device 23 through a second channel 24 to relieve a pain. The dosing devices 21, 23 can be e.g. infusion pumps, anesthesia vaporizers, or even manual injectors. At least one subject physiological parameter, which may be acquired through a signal line 25, is measured to observe the subject state such as an anesthesia state using a measurement unit 26 and a signal indicative of the at least one measured subject physiological parameter is created. The first dosing device 21 and the second dosing device 23 provide the information of the first drug and the second drug delivered to a processing unit 27 along signal lines 28, 29. The measurement unit 26 also provides a created signal indicative of the at least one measured subject physiological parameter and possibly the subject status to this processing unit 27 along a signal line 30. A user may give an additional information for the processing unit 27 regarding the subject demographic information and if possible an information about an operation using a user interface 31 through a communication line 32. The processing unit 27 after receiving the information from the user interface 31 and the signal from the measurement unit 26 and possibly the information from the first dosing device 21 and the second dosing device 23 determines a display information which is transformed through a signal line 33 to the display 1.

The received display information from the processing unit 27 is shown on the display 1 and which information includes a concentration of at least the first drug and the second drug as shown in Figure 1 and which is part of the arrangement of Figure 2. The display 1 shows two divergent axes including the first axis 2 and the second axis 3, one of these axes being for the variable first drug concentration and the other being for the variable second drug concentration. The optimal therapy mixture area 4 being part of the drug delivery information shown on the coordinate system comprises the first border section 5 determining a minimum concentration mixture for the first drug and the second drug and which optimal therapy mixture area 4 also comprises the second border section 6 determining a maximum concentration mixture for the first drug and the second drug. Various other features regarding the medical display 1 have already been explained hereinbefore while referring to Figure 1.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

**Claims**

1. A drug delivery presentation comprising:

    a display (1) for showing a concentration of at least two different types of drugs including a first drug and a second drug for a subject, and also for showing a coordinate system for a variable first drug concentration and a variable second drug concentration; and
    an optimal therapy mixture area (4) presented on a coordinate system comprising a first border section (5) determining a minimum concentration mixture for said first drug and said second drug,

    **characterized in that** said optimal therapy mixture area (4) further comprising a second border section (6) determining a maximum concentration mixture for said first drug and said second drug.

2. The drug delivery presentation according to claim 1, **characterized in that** said coordinate system is formed of a first axis (2) on said display (1) for a variable first drug concentration and a second axis (3) for a variable second drug concentration and which second axis is diverging from said first axis, whereupon the concentration values of both said first drug and second drug are configured to increase along said axes from an origin (10).

3. The drug delivery presentation according to claim 1, **characterized in that** said optimal therapy mixture area (4) is based on said subject demographic information and at least one known parallel physiological effect of said first drug and said second drug.

4. The drug delivery presentation according to claim 1, **characterized in that** said first drug is a hypnotic drug and said second drug is an analgesic drug.

5. The drug delivery presentation according to claim 1, **characterized in that** said optimal therapy mixture area (4) is based on at least one measured physiological parameter such as an electroencephalography, an electromyography, a plethysmography, a blood pressure or a heart rate.

6. The drug delivery presentation according to claim 4, **characterized in that** both said analgesic drug and said hypnotic drug have a hypnosis effect determined based on an electroencephalography signal and both said analgesic drug and said hypnotic drug have an analgesic effect determined based on one of a plethysmography signal, a blood pressure signal, a heart rate signal and an electromyography signal.

7. The drug delivery presentation according to claim 1, **characterized in that** initially when starting dosing said first drug and said second drug said optimal therapy mixture area (4) is based on said subject demographic information and at least one known parallel physiological effect of said first drug and said second drug, but thereafter when at least one measured subject physiological parameter is available, said optimal therapy mixture area (4) is updated based on this measured physiological parameter.

8. The drug delivery presentation according to claim 1, **characterized in that** a desired physiological effect is achieved with a higher concentration of said first drug and said second drug than defined by said first border section (5), but with a lower drug concentration than defined by said second border section (6).

9. The drug delivery presentation according to claim 1, **characterized in that** said first border section (5) determining a minimum concentration mixture is based on a formula:

$$E = \frac{E_{max} \cdot C^y}{C^y + EC_{50}{}^y}$$

where E is drug effect, $E_{max}$ is maximum drug effect, C is a drug concentration, y is a factor determining the slope of the sigmoidal rise, and $EC_{50}$ is the concentration providing 50% of the maximum effect.

10. The drug delivery presentation according to claim 1, **characterized in that** said second border section (6) determining a maximum concentration mixture is based on a formula:

$$E = \frac{E_{max} \cdot C^y}{C^y + EC_{50}{}^y}$$

where E is negative drug effect, $E_{max}$ is maximum drug effect, C is a drug concentration, y is a factor determining the slope of the sigmoidal rise of the negative effect, and $EC_{50}$ is the concentration providing 50% of the maximum negative effect.

11. The drug delivery presentation according to claim 1, further comprising at least one of a wake-up indicator (7) for timing of a subject wake-up, a drug cost indicator (8) showing the first and the second drug cost being acceptable and a current first and second drug delivery mixture indicator (9).

12. An arrangement for a drug delivery presentation, when dosing at least two different types of drugs including a first drug and a second drug for a subject, comprising:

   a first dosing device (21) for dosing said first drug;
   a second dosing device (23) for dosing said second drug;
   a measurement unit (26) for measuring at least one physiological parameter and creating a signal indicative of said at least one measured physiological parameter;
   a user interface (31) for entering a subject demographic information;
   a processing unit (27) for determining a display information based on said information received from said user interface (31) and said signal received from said measurement unit (26); and
   a display (1) for receiving said display information from said processing unit (27) for showing a concentration of at least said first drug and said second drug and for showing an optimal therapy mixture area (4) comprising a first border section (5) determining a minimum concentration mixture for said first drug and said second drug and which optimal therapy mixture area (4) is presented on a coordinate system,

   **characterized in that** said optimal therapy mixture area (4) further comprising a second border section (6) determining a maximum concentration mixture for said first drug and said second drug.

13. The arrangement according to claim 12, **characterized in that** said optimal therapy mixture area (4) is based on at least one measured subject physiological parameter such as an electroencephalography, an electromyography, a plethysmography, a blood pressure or a heart rate.

14. The arrangement according to claim 12, **characterized in that** said first border section (5) determining a minimum concentration mixture is based on a formula:

$$E = \frac{E_{max} \cdot C^y}{C^y + EC_{50}{}^y}$$

where E is drug effect, $E_{max}$ is maximum drug effect, C is a drug concentration, y is a factor determining the slope of the sigmoidal rise, and $EC_{50}$ is the concentration providing 50% of the maximum effect.

15. The arrangement according to claim 12, **characterized in that** said second border section (6) determining a maximum concentration mixture for said first drug and said second drug is based on a formula:

$$E = \frac{E_{max} \cdot C^y}{C^y + EC_{50}{}^y}$$

where E is negative drug effect, $E_{max}$ is maximum drug effect, C is a drug concentration, y is a factor determining the slope of the sigmoidal rise of the negative effect, and $EC_{50}$ is the concentration providing 50% of the maximum negative effect.

Figure 1

Figure 2

**PARTIAL EUROPEAN SEARCH REPORT**   Application Number

which under Rule 63 of the European Patent Convention EP 08 39 6017
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | THOMAS W. BOUILLON ET AL.: "Pharmacodynamic Interaction between Propofol and Remifentanil Regarding Hypnosis, Tolerance of Laryngoscopy, Bispectral Index, and Electroencephalographic Approximate Entropy" ANESTHESIOLOGY, vol. 100, no. 6, June 2004 (2004-06), pages 1353-1372, XP002529448 * the whole document * | 12-15 | INV. G06F19/00 |
| Y | THOMAS W. BOUILLON, PETER M. SCHUMACHER: "Anesthesia Advisory Display" PROCEEDINGS OF AMCA 2005: ADVANCED MODELING AND CONTROL IN ANESTHESIA, [Online] 12 April 2005 (2005-04-12), - 14 April 2005 (2005-04-14) pages 1-19, XP002529449 Retrieved from the Internet: URL:http://www.amca2005.unibe.ch/kas/pres_pdf/20050412_4b_BoullionThomas.pdf> [retrieved on 2009-05-15] * page 1 - page 19 * | 12-15 | |

TECHNICAL FIELDS SEARCHED (IPC)

G06F

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 26 May 2009 | Chabros, Cezary |

Europäisches
Patentamt

European
Patent Office

Office européen
des brevets

Claim(s) searched completely:
        12-15

Claim(s) not searched:
        1-11

Reason for the limitation of the search (non-patentable invention(s)):

Excluded under Article 52(2)(d) EPC: presentation of information.